# EUROPEAN PATENT SPECIFICATION

(11) **EP 4 317 158 B1**
(45) Date of publication and mention of the grant of the patent: **11.11.2026**
(21) Application number: 22778836.1
(22) Date of filing: 28.03.2022
(51) Int. Cl.: C07D 471/04, A61P 29/00, A61P 25/04, A61P 1/16, A61P 35/00, A61P 9/10, A61P 9/00, A61P 25/08, A61P 25/06, A61K 31/4745

(54) **QUINONE COMPOUND AND PHARMACEUTICAL USE THEREOF**
CHINONVERBINDUNG UND IHRE PHARMAZEUTISCHE VERWENDUNG
COMPOSÉ QUINONE ET SON UTILISATION PHARMACEUTIQUE

(30) Priority: 02.04.2021 CN 202110364689
(43) Date of publication of application: 07.02.2024
(73) Proprietor: Nanjing Shupeng Life Science Co., Ltd., Nanjing, Jiangsu 210000 (CN)
(72) Inventor: WEN, Chuanjun, Nanjing, Jiangsu 210000 (CN); ZOU, Junyi, Nanjing, Jiangsu 210000 (CN); SUN, Fenyong, Nanjing, Jiangsu 210000 (CN)
(74) Representative: karo IP
(86) International application number: PCT/CN2022/083261
(87) International publication number: WO 2022/206654

(56) References cited:
- EP-A1- 3 199 154
- CN-A- 104 856 996
- CN-A- 107 007 606
- SAIHARA KAZUHIRO, KAMIKUBO RYOSUKE, IKEMOTO KAZUTO, UCHIDA KOJI, AKAGAWA MITSUGU: "Pyrroloquinoline Quinone, a Redox-Active o -Quinone, Stimulates Mitochondrial Biogenesis by Activating the SIRT1/PGC-1α Signaling Pathway", BIOCHEMISTRY, vol. 56, no. 50, 19 December 2017 (2017-12-19), pages 6615 - 6625, XP055974716, ISSN: 0006-2960, DOI: 10.1021/acs.biochem.7b01185
- CHENG, QIONG ET AL.: "Pyrroloquinoline quinone promotes mitochondrial biogenesis in rotenone-induced Parkinson's disease model via AMPK activation", ACTA PHARMACOLOGICA SINICA, vol. 42, no. 5, 28 August 2020 (2020-08-28), XP037430566, ISSN: 1671-4083, DOI: 10.1038/s41401-020-0487-2
- YANG, CHONGFEI ET AL.: "Pyrroloquinoline Quinone (PQQ) Inhibits Lipopolysaccharide Induced Inflammation in Part via Downregulated NF-kB and p38/JNK Activation in Microglial and Attenuates Microglia Activation in Lipopolysaccharide Treatment Mice", PLOS ONE, vol. 9, no. 10, 14 October 2014 (2014-10-14), XP055823298, ISSN: 1932-6203, DOI: 10.1371/journal.pone.0109502

## Description

### TECHNICAL FIELD

The present invention relates to the field of medical chemistry, and specifically relates to a novel structural compound having a chemical structure of a general formula (I).

### BACKGROUND

Pyrroloquinoline quinone (PQQ), as a third prosthetic group found in bacterial dehydrogenases after flavin nucleotide (FAD) and nicotinamide nucleotide (NAD), has a chemical name of 4,5-dihydro-4,5-dioxo-1H-pyrrolo(2,3-f)quinone-2,7,9-tricarboxylic acid. The PQQ has a structural formula as follows:

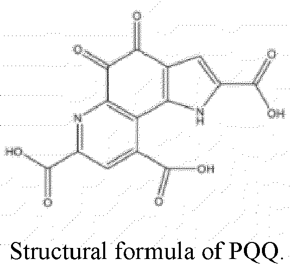

Although the PQQ can be synthesized by only few bacteria under natural conditions, a trace amount of the PQQ has been found in various plants, animals and human bodies. In particular, the PQQ has a high relative content in breast milk. As an oxidoreductase prosthetic group generally found in animal and plant tissues, the PQQ has a very low content and important effects, which not only is a newly discovered component of a mitochondrial respiratory chain for participating in catalysis of redox reactions in organisms, but also has extremely diverse biological activities, and many obstacles will be caused to mammals by deficiency of the PQQ. Thus, the PQQ has been considered as a novel vitamin.

Research on the PQQ can be traced back to 1959 when it was observed in studying glucose metabolism of non-phosphorylated bacteria (glucose metabolism of bacteria was generally realized through a glucose-6-phosphate way) that Acinetobacter calcoaceticus contains a glucose dehydrogenase (GDH) independent of the NAD (P) and the FAD. Then, Hauge obtained a separable prosthetic group from such enzyme and inferred that the prosthetic group might be a naphthoquinone derivative.[Hauge, J.G., Glucose dehydrogenase of bacterium anitratum: an enzyme with a novel prosthetic group. J Biol Chem. 1964 Nov;
Duine et al. suggested in 1979 that the prosthetic group was a quinone structural substance containing two N atoms [Duine JA, Frank J, van Zeeland JK. Glucose dehydrogenase from Acinetobacter calcoaceticus: a 'quinoprotein'. FEBS Lett. 1979 Dec 15; 108 (2): 443-6.]. Meanwhile, after analyzing a crystalline acetone adjunct of the prosthetic group by an X-ray crystal diffraction technology, Salisbury et al. determined that the prosthetic group was of a tricarboxypyrroliquinoline quinone structure and had a structural formula of 4,5-dihydro-4,5-dioxo-1H-pyrrolo(2,3-f)quinone-2,7,9-tricarboxylic acid [Salisbury SA, Forrest HS, Cruse WB, Kennard O. Nature. 1979 Aug 30; 280 (5725): 843-4].

Discovery of the PQQ is an important event in research history of quinone enzymes. The PQQ not only enables people to know a new prosthetic group, but also indicates that the enzymology will have a new branch: quinone enzymes, namely, oxidoreductases with the PQQ and other quinone compounds as prosthetic groups. Before the discovery of the PQQ, people believed that the oxidoreductases had only two types of prosthetic groups, namely NAD/NADP and FAD/FMN.

The PQQ has a variety of functions, which are briefly introduced as follows.

### 1. A variety of nutrition and nourishing functions of the PQQ

The PQQ can promote growth of microorganisms and plants and affect growth and reproduction of animals. The PQQ is an essential nutritional factor as it cannot be produced endogenously in animals. Poor growth and decreased fertility of mice will be caused by deficiency of the PQQ. The litter number of female mice is only half of that of female mice having a PQQ-containing diet, and half of offsprings of the PQQ-deficient female mice are dead before a weaning period (4 weeks).

When the content of the PQQ in food of mice is lower than 200 ng/g, cytokinin required for cell proliferation, especially IL-2 for promoting cell division and proliferation of lymphocytes, is decreased, maturation and processing of T-cells are hindered, and finally mature T-cells have defects, leading to low immune responses of the mice [Steinberg, F.M. et al. Dietary pyrroloquinoline quinone: growth and immune response in BALB/c mice. Journal of Nutrition, 1994. 124 (5): p 744].

When pregnant mice or newborn mice are in deficiency of the PQQ, the content of lysyloxidase in the skin is obviously decreased (only 10-30% of a normal skin value), the solubility of collagen is doubled, and the skin becomes dry and tends to fall off. It is indicated that the PQQ has a great value in collagen crosslinking and skin health [Nishigori et al, Preventive effect of pyrroloquinoline quinone (PQQ) on biliverdin accumulation of the liver of chick embryo after glucocorticoid administration. Life Sciences, 1993. 52 (3): p 305-12.].

### 2. Antioxidant function

The production and removal of various free radicals in organisms stay in a dynamic balance state, and that is to say, free radicals are always maintained at a certain level in the body. Too many or too few free radicals are unfavorable for the body. When too many free radicals are contained, aging of the body is promoted, leading to various diseases, including cancers, heart diseases and the like. When too few free radicals are contained, health is also affected, and even normal metabolism is hindered or another disease is induced. The PQQ prevents oxidative damage of the body through the following several mechanisms.

Free PQQ exists in cell tissues and body fluids of animals in a quinone type, a hydroquinone type and a semi-quinone type, which can catalyze mutual conversion of oxygen and O₂-, so as to keep free radicals maintained in a balance state in the body.

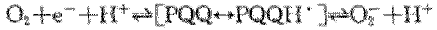

The PQQ binds to superoxide dismutase (SOD) to form a broad-spectrum oxidase system for producing hydrogen peroxide, so as to inhibit superoxide anion free radicals from damaging cells: the SOD is used as an apoenzyme protein, and the PQQ is used as a non-covalently bound redox prosthetic group.

The PQQ converts oxidized glutathione (GSSG) into reduced glutathione (GSH) more rapidly by accelerating an "NAD+→NADH" reaction.

2O₂ +2e˙ → 2O*ᵢ*

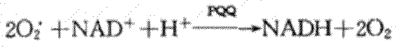

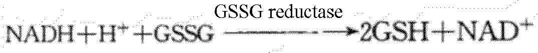

The PQQ also has a strong ability to remove the free radicals, which is equivalent to 50-100 times of that of ascorbic acid [Akaike T, Sato K, Maeda H, et al. PQQ as a Generator and a Scavenger of Oxygen Radicals: Determination with ESR Spectroscopy using a Spin Trap Agent [M]// Enzymes Dependent on Pyridoxal Phosphate and Other Carbonyl Compounds As Cofactors. 1991.].

### 3. PQQ and diseases

Because of nutritional and antioxidant characteristics, the PQQ has shown certain preventive and therapeutic effects in a variety of laboratory disease models.

### (1) Protection of the heart from oxidative damage

A protective effect of the PQQ on the heart is related to the ability to scavenge free radicals. The PQQ can scavenge reactive oxygen species (ROS) generated by hypoxia-reperfusion and significantly reduce the release of lactic dehydrogenase in the heart, and under the catalytic action of flavin reductase, a catalytic product can also reduce a peroxidation state of hemoglobin and eliminate myocardial damage caused by the hypoxia-reperfusion. Studies have shown that by using the PQQ to protect hearts of ischemia-reperfusion mice, the extent of myocardial infarction is obviously lowered, the pressure of the ventriculus sinister and the ascending and descending rates of the diastolic blood pressure of the ventriculus sinister are increased, ventricular fibrillation is reduced, and the level of malondialdehyde in myocardial tissues is lowered. The PQQ can also inhibit the generation of hydrogen peroxide-induced ROS in cardiomyocytes of rats and the decrease of mitochondrial membrane potential, thereby reducing oxidative stress, inhibiting inactivation of mitochondrial functions and protecting the cardiomyocytes of the rats [Zhu BQ, Zhou HZ, Teerlink JR, Karliner JS. Cardiovasc Drugs Ther. 2004 Nov; 18(6):421-31; Xu X, Chen C, Lu WJ, Su YL, Shi JY, Liu YC, Wang L, Xiao CX, Wu X, Lu Q. Cardiovasc Diagn Ther. 2020 Jun; 10 (3): 453-469.].

### (2) Prevention and treatment of liver injuries

Experimental liver injuries of rats caused by carbon tetrachloride (CCl), galactosamine, acetamide sulfide and other toxins can be prevented by intraperitoneal injection of a certain dose of the PQQ and derivatives thereof in advance. The PQQ can reduce the generation of the ROS induced by toxic substances of the liver, obviously reduce levels of glutamic pyruvic transaminese (GPT) and lactic dehydrogenase in serum bilirubin and hinder necrosis of liver cells without affecting conventional biochemical indicators of rats (such as blood glucose, blood urea nitrogen). [Tsuchida, T., et al., The protective effect of pyrroloquinoline quinone and its derivatives against carbon tetrachloride-induced liver injury of rats. J Gastroenterol Hepatol, 2010. 8 (4): p. 342-347.].

### (3) Promotion of nerve growth and protection of the nervous system

As a first discovered and most thoroughly studied one among neurotrophic factors, a nerve growth factor (NGF) has dual biological functions of neuronal nutrition and neuroprotection, which plays an important regulatory role in growth, development, differentiation, regeneration and specific expression of biological functions of central and peripheral neurons. Experiments have shown that outside the body, the PQQ can stimulate L-M cells and Schwann cells to generate the NGF [Urakami T, Tanaka A, Yamaguchi K, et al. Synthesis of esters of coenzyme PQQ and IPQ, and stimulation of nerve growth factor production. [J]. Biofactors, 1995, 5 (3): 139.].

### (4) Prevention of acetaldehyde poisoning

As an intermediate metabolite of alcohol in animals, acetaldehyde is toxic. Due to gene mutation and insufficient function of an acetaldehyde dehydrogenase, many people have accumulation of the acetaldehyde after drinking, leading to slight acetaldehyde poisoning responses such as red faces and ears and dizziness.

Experiments using rodents have found that the PQQ is conducive to metabolism of the acetaldehyde. Before administered with ethanol by gavage, mice were administered with the PQQ (at a dose of 11.5 mg/kg body weight) by intraperitoneal injection. The test has found that the mice in a treatment group and a control group have no obvious differences in the concentration of ethanol in the blood and liver, but the former group has a much lower concentration of acetaldehyde than the latter group. When the PQQ is replaced with other quinone derivatives such as a coenzyme Q10, similar effects are also achieved [Hobara N, Watanab A, Kobayashi M, et al. Quinone derivatives lower blood and liver acetaldehyde but not ethanol concentrations following ethanol loading to rats. [J]. Pharmacology, 1988, 37 (4): 264-267.].

### (5) Analgesic effect

Neuropathic pain is chronic pain caused by damage to a somatosensory nervous system. Gong et al. found that the PQQ had an analgesic effect in a chronic constriction injury (CCI) model of rats, which might be associated with an inhibition effect of an N-methyl-D-aspartic acid (NMDA) receptor of the PQQ [Gong D, Geng C, Jiang L, Aoki Y, Nakano M, Zhong L. Effect of pyrroloquinoline quinone on europathic pain following chronic constriction injury of the sciatic nerve in rats. Eur J Pharmacol. 2012 Dec 15; 697 (1-3): 53-8.].

A first PQQ dietary supplement was approved by the United States in 2009. As the PQQ is slightly soluble in water, the supplement includes a PQQ sodium salt (PQQ-2NA+) as an ingredient and has better solubility. In 2018, the PQQ-2Na+ was also approved by the European Union as a healthy food, which is suitable for adults except pregnant women and breastfeeding women. The dose of the PQQ sodium salt on the market is equal to or less than 20 mg/day. The PQQ mainly absorbed by the small intestines (about 60%) after oral administration and then excreted by the kidney (through urine) by 80% 24 hours after the administration. The safe dose for rats is as shown in the table below.

### Animal safety experiment of rats after oral administration of PQQ

| Gender | Duration of oral administration/day | Oral dose of rats (mg/kg/day) | | Adverse response | |
|---|---|---|---|---|---|
| Male | 14 | Median lethal dose (LD50) | 1,000-2,000 | None | |
| Female | 14 | | 500-1,000 | Reversible adverse response | Increased kidney weight |
| | | | | | Increased urine proteins and crystals |
| Male | 91 | 400 | | None | |
| Female | 91 | 400 | | None | |

In a controlled double-blind clinical trial of humans, volunteers have no adverse responses and no changes of kidney injury markers after taking the PQQ at 60 mg/day for one month. According to a safe dose determined by the Food and Drug Administration (FDA), healthy adults with a body weight of about 60 kg rarely have adverse events after taking 240 mg of the PQQ every day. FDA, Generally Recognized as Safe (GRAS) notice of pyrroloquinoline quinone disodium salt as a food ingredient.

The PQQ has been discovered for more than 40 years and used as a health care product in the United States for 10 years, but has never been developed and used as a drug. It is believed that the following three reasons may be acceptable: 1, a PQQ prototype has low bioavailability and low water solubility, and studies have found that the PQQ has a bioavailability of about 12% in beagles; 2, the PQQ has unclear targets and unclear molecular mechanisms; and 3, as the structure of the PQQ has been disclosed for more than 40 years without protection, the PQQ has a limited commercial value in drug development. Therefore, a novel structure with higher bioavailability, clear targets, clear molecular mechanisms, obvious efficacy and patent protection obtained through structural modification of the PQQ has great significance in both the scientific industry and the commercial industry.

AMP-activated protein kinase (AMPK) is a key molecule in metabolic regulation of biological energy and is the core of studying diabetes mellitus and other metabolism-related diseases. The AMPK is expressed in a variety of metabolism-related organs and can be activated by a variety of stimuli in the body, including cell stress, cell movement and many hormones as well as substances that can affect cell metabolism. Genetic and pharmacological studies have shown that the AMPK is necessary for the body to maintain glucose balance. In addition to being associated with metabolic diseases, the AMPK is also associated with neurodegenerative diseases [Curry DW, Stutz B, Andrews ZB, Elsworth JD. Targeting AMPK Signaling as a Neuroprotective Strategy in Parkinson's Disease. J Parkinsons Dis. 2018; 8 (2): 161-181.], cardiovascular diseases [Feng Y, Zhang Y, Xiao H. AMPK and cardiac remodelling. Sci China Life Sci. 2018 Jan; 61 (1): 14-23.], tumors [Wang Z, Wang N, Liu P, Xie X. AMPK and Cancer. Exp Suppl. 2016; 107: 203-226.], neuropathic pain [Asiedu MN, Dussor G, Price TJ. Targeting AMPK for the Alleviation of Pathological Pain. Exp Suppl. 2016; 107: 257-285.], pathogenic microorganism infection (including viruses) [Silwal P, Kim JK, Yuk JM, Jo EK. AMP-Activated Protein Kinase and Host Defense against Infection. Int J Mol Sci. 2018 Nov 6; 19 (11): 3495.] and other diseases. The AMPK is also an important target for research on delaying of aging and prolonging of life [Burkewitz K, Zhang Y, Mair WB.AMPK at the nexus of energetics and aging. Cell Metab. 2014 Jul 1; 20 (1): 10-25.]. Cheng et al. reported that in a rotenone-induced Parkinson's disease model, the PQQ can promote mitochondrial synthesis by activating the AMPK, thereby having a therapeutic effect on diseases [Cheng Q, Chen J, Guo H, Lu JL, Zhou J, Guo XY, Shi Y, Zhang Y, Yu S, Zhang Q, Ding F. Pyrroloquinoline quinone promotes mitochondrial biogenesis in rotenone-induced Parkinson's disease model via AMPK activation. Acta Pharmacol Sin. 2020 Aug 28]. It has been found in a variety of cells that 100 nM PQQ and structurally modified compounds thereof, SP3101 and SP3102, can significantly up-regulate the phosphorylation level of the AMPK (the activation state of the AMPK).

It is generally believed that the PQQ achieves a series of biological effects by stimulating antioxidant signaling pathways in the body. The PQQ has a slower effect when used as an analgesic drug. Therefore, the PQQ is not suitable for use as an acute analgesic drug.

### SUMMARY

The purpose of the present invention is to obtain a compound of a structural formula (I) by modifying the structure of PQQ. The compound has the characteristics of a rapid analgesic effect and a long-acting analgesic effect.

The references to methods of treatment in the subsequent paragraphs of this description are to be interpreted as references to the compounds, pharmaceutical compositions and medicaments of the present invention for use in a method for treatment of the human (or animal) body by therapy (or for diagnosis).

On the one hand, the present invention provides a compound of a formula (I) or a pharmaceutically acceptable salt, ester, stereoisomer, or solvent compound thereof, 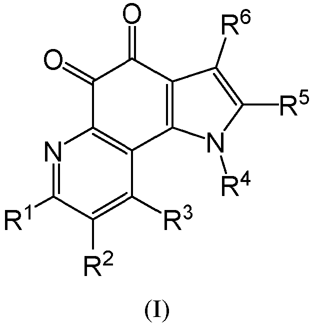 where
R¹ is selected from -OH, -OR, -OC(O)R, -NH₂, -NHR, -SH or -SR;
R² is selected from H,;
R³ is selected from H, or -COOH
R⁴ is selected from H;
R⁵ is selected from H, or -COOH;
R⁶ is selected from H;
R is C₁-C₆ alkyl; and in some specific examples, the R is C₁-C₃ alkyl.

In some examples of the present invention, the present invention further provides a compound of a formula (II) or a pharmaceutically acceptable salt, ester, stereoisomer, or solvent compound thereof, 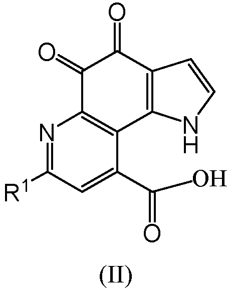 where R¹ is selected from -OH, -OR, -OC(O)R, -NH₂, -NHR, -SH or -SR; R is C₁-C₆ alkyl; and in some specific examples, the R is C₁-C₃ alkyl.

The present invention further provides some compounds or pharmaceutically acceptable salts, esters, stereoisomers, or solvent compounds thereof shown as follows:

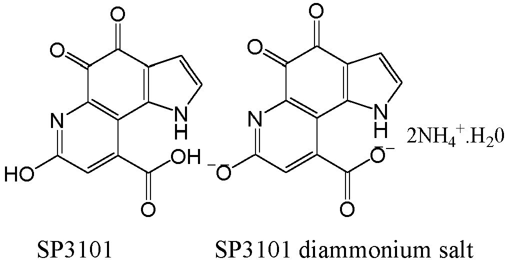

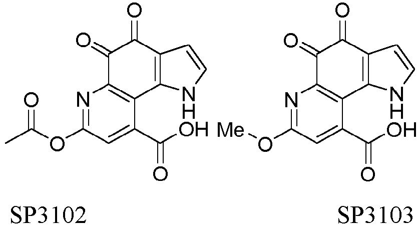

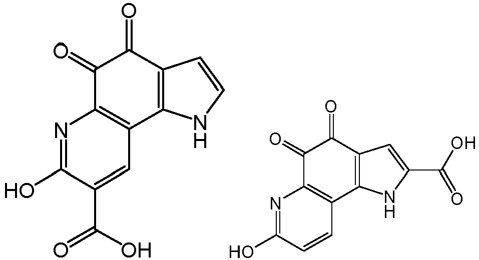

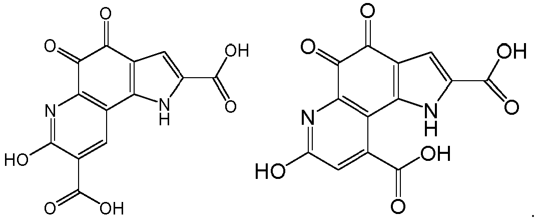

On the other hand, the present invention further provides a method for preparing the compound of a formula (II):

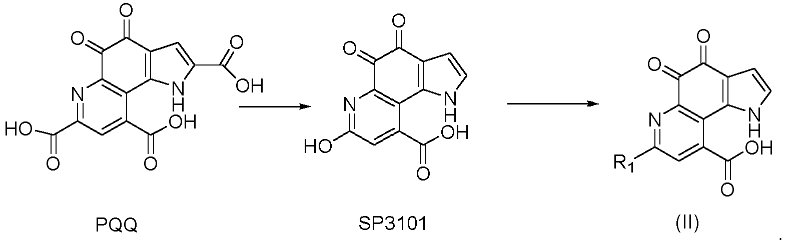

Salts that may be formed by the compounds of the present invention also fall within the scope of the present invention. Unless otherwise specified, the compounds of the present invention are understood as including their salts. For example, the compound of a formula (I) reacts with a certain amount, such as an equivalent amount, of an acid or an alkali, followed by salting out in a medium or freeze-drying in an aqueous solution.

The compounds of the present invention, which are obtained by sequentially performing preparation, separation and purification, have a weight content of equal to or greater than 90%, such as equal to or greater than 95% and equal to or greater than 99% ("extremely pure" compounds), as described and listed herein. Herein, the "extremely pure" compounds of the present invention are also a part of the present invention.

The present invention further provides a pharmaceutical composition. The pharmaceutical composition includes the compounds of the present invention or pharmaceutically acceptable salts, esters, stereoisomers, or solvent compounds thereof, and a pharmaceutically acceptable carrier.

The present invention further provides use of the compounds of the present invention or pharmaceutically acceptable salts, esters, stereoisomers, or solvent compounds thereof as an AMPK agonist.

The present invention further provides use of the compounds of the present invention or pharmaceutically acceptable salts, esters, stereoisomers, or solvent compounds thereof as an inhibitor of a Cox-2 enzyme.

The present invention further provides the compounds of the present invention or pharmaceutically acceptable salts, esters, stereoisomers, or solvent compounds thereof for use asanalgesic drugs.

The analgesic drugs of the present invention can be used for relieving pain, which may be any uncomfortable feeling caused by physical injuries, diseases or adverse external stimulus, such as various kinds of pain caused by surgery, inflammations, tumors, myocardial infarction injuries, bacterial infection, viral infection, neurological diseases and other various factors; and the pain caused by neurological diseases, for example, includes but is not limited to pain caused by neurodegeneration and neurological changes, or pain caused by epilepsy, migraine and the like, or pain caused by various liver injuries, myocardial infarction injuries and the like.

The present invention further provides the compounds of the present invention or pharmaceutically acceptable salts, esters, stereoisomers, or solvent compounds thereof for use as drugs for prevention and treatment of inflammatory diseases; and the inflammatory diseases of the present invention may be inflammatory responses caused by bacterial or viral infection, for example, include but are not limited to pulmonary inflammations and respiratory tract inflammations caused by bacterial or viral infection, viral myocarditis and hepatitis or hepatic insufficiency caused by bacteria or viruses, may also be excessive inflammation responses caused by tumor immunotherapy, and may also be inflammation responses caused by autoimmune diseases, allogeneic organs and rejection responses of tissue transplantation.

The present invention further provides the compounds of the present invention or pharmaceutically acceptable salts, esters, stereoisomers, or solvent compounds thereof for use as anti-tumor drugs.

The present invention further provides the compounds of the present invention or pharmaceutically acceptable salts, esters, stereoisomers, or solvent compounds thereof for use in chemotherapeutic treatment, chemical substances or drugs for prevention and treatment of tumors caused by cytotoxins, drugs for prevention and treatment of various liver injuries and hepatic insufficiency caused by chronic hepatitis, and drugs for prevention and treatment of myocardial infarction injuries and viral myocarditis.

The present invention further provides the compounds or pharmaceutically acceptable salts, esters, stereoisomers, or solvent compounds thereof for use as drugs for prevention and treatment of neurodegeneration, change diseases, epilepsy and migraine.

The present invention further provides the compounds of the present invention or pharmaceutically acceptable salts, esters, stereoisomers, or solvent compounds thereof for use as drugs for prevention and treatment of viral infection and inflammatory storms caused by viral infection.

The present invention further provides the compounds of the present invention or pharmaceutically acceptable salts, esters, stereoisomers, or solvent compounds thereof for use as drugs for prevention and treatment of peripheral neuropathy caused by chemotherapy, such as pain caused by chemotherapy.

The present invention further provides the compounds of the present invention or pharmaceutically acceptable salts, esters, stereoisomers, or solvent compounds thereof for use as drugs for prevention and treatment of depression, and sedative or hypnotic drugs.

Unless otherwise specified, the terms of the present invention are defined as follows.

The terms "C₁-C₃ alkyl", "C₁-C₆ alkyl", "C₂-C₆ alkyl" and "C₃-C₆ alkyl" refer to saturated straight-chain or branched-chain monovalent hydrocarbon groups containing one to three, one to six, two to six, or three to six carbon atoms, respectively. Instances include (but are not limited to) methyl, ethyl, 1-propyl, isopropyl, 1-butyl, isobutyl, sec-butyl, tert-butyl, 2-methyl-2-propyl, pentyl, neopentyl and hexyl. The alkyl may be substituted or unsubstituted. When substituted, the alkyl is preferably substituted with one or more substituents, more preferably, one, two or three substituents, and further preferably, one or two substituents, and the substituent is independently selected from lower alkyl, trihaloalkyl, halogen, hydroxyl, lower alkoxyl, sulfhydryl, (lower alkyl) sulfonyl, cyano, acyl, thioacyl, O-carbamoyl, N-carbamoyl and the like.

The term "aryl" refers to an all-carbon monocyclic group or a fused polycyclic group containing 1 to 12 carbon atoms with a fully conjugated π-electron system. Non-restrictive instances of the aryl include phenyl, naphthyl and anthryl. The aryl may be substituted or unsubstituted. When substituted, the aryl is preferably substituted with one or more substituents, more preferably, one, two or three substituents, and further preferably, one or two substituents, and the substituent is independently selected from lower alkyl, trihaloalkyl, halogen, hydroxyl, lower alkoxyl, sulfhydryl, (lower alkyl) sulfonyl, cyano, acyl, thioacyl, O-carbamoyl, N-carbamoyl, O-thiocarbamoyl, N-thiocarbamoyl, C-acylamino, N-acylamino, nitro, N-sulfonamido, S-sulfonamido and the like. Preferably, the aryl is optionally substituted with one or two substituents, and the substituent is independently selected from halogen, lower alkyl, trihaloalkyl, hydroxyl, sulfhydryl, cyano, N-acylamino, monoalkylamino or dialkylamino, carboxyl or N-sulfonamido.

Compared with the prior art, the present invention has the following beneficial effects.

The compounds of the present invention have good water solubility and high bioavailability after salification. Biologically, these novel structures not only have an AMPK activation effect, but also unexpectedly have an inhibition effect on a Cox-2 enzyme, and have unique anti-inflammatory and analgesic effects. In an inflammatory pain model established by using a complete Freund's adjuvant, single doses of SP3101, SP3102 and SP3103 have a rapid analgesic effect and an obvious analgesic effect 1.5 hours after oral administration, and the analgesic effect can last for 72 hours. Interestingly, when an AMPK inhibitor is injected into caudal veins of rats half an hour before administration, a short-acting analgesic effect of these analgesics is not affected, but the analgesic effect is completely eliminated after 24 hours, indicating that a long-acting analgesic effect is dependent on activation of AMPK. It is also indicated that such drugs have analgesic effects through at least two targets, namely, different targets for achieving a short-acting (1.5 hours) analgesic effect and a long-acting (24-72 hours) analgesic effect.

### BRIEF DESCRIPTION OF THE DRAWINGS

FIG. 1 shows an analgesic effect of SP3101 on rats after single oral administration at a dose of 20 mg/kg;
FIG. 2 shows an analgesic effect of ibuprofen on rats after continuous oral administration at a dose of 25 mg/kg;
FIG. 3 shows an analgesic effect of SP330 (PQQ) on rats after continuous oral administration at a dose of 20 mg/kg/day for three days;
FIG. 4 shows a long-acting analgesic effect of SP3101 inhibited by a compound c as an AMPK inhibitor;
FIG. 5 shows molecular action mechanisms of SP3101 series molecules;
FIG. 6 shows an effect of SP3101 on inhibiting the activity of cox-2;
FIG. 7 shows an effect of SP3101 on inhibiting a lung inflammation of mice induced by a mouse hepatitis virus (MHV);
FIG. 8 shows an analgesic effect of SP3101 on mice using a chronic constriction injury (CCI) model of the sciatic nerve after single oral administration at a dose of 40 mg/kg;
FIG. 9 shows sedation test results of SP3101;
FIG. 10 shows test effects of SP3101 using an oxaliplatin chemotherapy pain model;
FIG. 11 shows statistics of the percentage of positive responses of mice in a total detection number after selecting Von Frey fibrils with a specific pain threshold of 1.0 g, detecting each mouse for 10 times and recording the positive response frequency of the mice;
FIG. 12 shows statistics of the percentage of positive responses of mice in a total detection number after selecting Von Frey fibrils with a specific pain threshold of 0.4 g, detecting each mouse for 10 times and recording the positive response frequency of the mice;
FIG. 13 shows statistics of the percentage of positive responses of mice in a total detection number after selecting Von Frey fibrils with a pain threshold of 0.07 g, detecting each mouse for 10 times and recording the positive response frequency of the mice; and
FIG. 14 shows statistics of the percentage of positive responses of mice in a total detection number after selecting Von Frey fibrils with a pain threshold of 0.4 g, detecting each mouse for 10 times and recording the positive response frequency of the mice.

### DETAILED DESCRIPTION

The following examples are described to facilitate a better understanding of the present invention and are not intended to limit the present invention. Unless otherwise specified, experimental methods described in the following examples are conventional methods. Unless otherwise specified, test materials used in the following examples are purchased from conventional biochemical reagent stores.
1. Instruments and reagents: An ALMEMO2490 data collector, purchased from AHLBORN company in Germany;
2. A PQQ disodium salt, purchased from Hisun Pharmaceutical;
3. An MHV-A59 virus strain, purchased from Suzhou Xishan Biotechnology Co., Ltd., at a plaque forming unit (PFU)/ml of 1.4×10⁷;
4. RAW264.7, purchased from Peking Union Cell Resource Center.

### Example 1 Preparation and identification of a compound SP3101 (Chinese name: 4,5-dioxo-4,5-dihydro-1H-pyrrolo[2,3-f]quinone-7-hydroxy-9-carboxylic acid)

A PQQ sample was dissolved in a 1 M NaOH aqueous solution with a concentration of 100 mg/ml, loaded into several reaction bottles to carry out a reaction under microwave conditions at 250°C for 1 hour, then taken out and detected by liquid chromatography-mass spectrometry (LCMS) to obtain a main degradation product with a molecular weight of 258. Then, the sample was filtered and prepared for several times by preparative liquid chromatography (Prep-LC) at high pressure under the following preparation conditions: high pressure C18-acetonitrile/2 mM ammonium acetate at a gradient of 2-30%. After spin-dried to remove an organic phase, the prepared sample was freeze-dried, and the freeze-dried sample was dried in an oven at 105°C for 2 hours and then weighed.

According to detection by mass spectrometry-electrospray in a positive ion mode, the sample has a mass-to-charge ratio (m/z) of 259.2 (M+1)+, proving that the compound has a free molecular weight of 258.

Nuclear magnetic resonance spectra are as shown in Table 1.

**Table 1 Attribution of hydrogen spectra and carbon spectra**

| Number | ¹HNMR | ¹³CNM^{R} |
|---|---|---|
| 1 | 6.24 d, 1H, *j*=2.4 | 105.4 |
| 2 | 6.91 d, 1H, *j*=2.4 | 126.7 |
| 3 | / | 124.4 |
| 4 | / | 184.9 |
| 5 | / | 159.9 |
| 6 | / | 136.2 |
| 7 | / | 165.9 |
| 8 | 8.15, 1H, s | 128.4 |
| 9 | / | 147.1 |
| 10 | / | 133.1 |
| 11 | / | 149.7 |
| 12 | / | 167.0 |

| | | |
|---|---|---|
| Note: The sample was dissolved in DMSO-d6. | | |

After nuclear magnetic data were compared and analyzed, maleic acid was added into the sample and then detected to obtain hydrogen spectra. Two NH4⁺ signals can be clearly shown, indicating that the compound is a diammonium salt.

Results of weight loss analysis using a thermal gravimetric analyzer (TGA) show that the sample has weight loss of 5.97% at 150°C. Combined with the previous drying of the sample at 105°C and nuclear magnetic resonance, it is inferred that the sample contains one molecule of bound water.

According to the analysis of a purified impurity by ultrahigh performance liquid chromatography-mass spectrometry (UPLC-MS), the analysis of the nuclear magnetic spectra and the weight loss analysis by a TGA, it can be determined that the compound is of the following structure, is named an SP3101 diammonium salt and can be converted into an SP3101 form through conventional treatment.

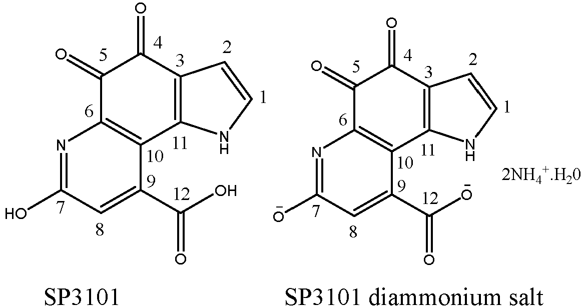

### Example 2 Preparation and identification of a compound SP3102 (Chinese name: 7-acetoxy-4,5-dioxo-4,5-dihydro-1H-pyrrolo[2,3-f]quinone-9-carboxylic acid)

A raw material SP3101 (3.1 g, 0.01 mole) was dissolved in dry pyridine (6 ml), then acetic anhydride (6 ml) was added and stirred to carry out a reaction at room temperature for 20 hours. After the reaction was completed, a solvent was spin-dried, and a resulting solid was separated by column chromatography to obtain a compound SP3102.

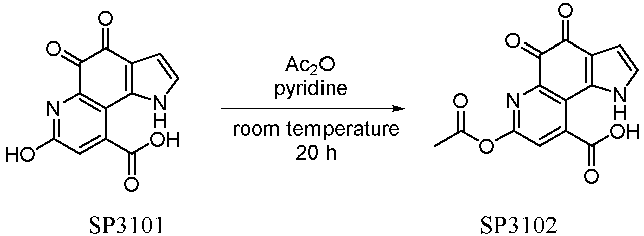

According to detection by mass spectrometry-electrospray in a positive ion mode, the sample has an m/z value of 300.1 (M+1)+, proving that the compound SP3102 has a free molecular weight of 300. Nuclear magnetic resonance spectra are as shown in Table 2.

**Table 2 Attribution of hydrogen spectra and carbon spectra**

| Number | ¹H NMR | ¹³C NMR |
|---|---|---|
| 1 | 2.28, 3H, s | 20.3 |
| 2 | 5.0, 1H, s | / |
| 3 | 6.40 d, 1H, *j*=7.5 | 104.4 |
| 4 | 6.95 d, 1H, *j*=7.5 | 118.3 |
| 5 | 7.82, 1H, s | 112.9 |
| 6 | 11.0, 1H, s | / |
| 7 | / | 122.7 |
| 8 | / | 125.2 |
| 9 | / | 126.4 |
| 10 | / | 137.2 |
| 11 | / | 144.8 |
| 12 | / | 156.4 |
| 13 | / | 167.7 |
| 14 | / | 172.6 |
| 15 | / | 173.5 |
| 16 | / | 176.7 |

The sample was dissolved in DMSO-d6.

### Example 3 Preparation and identification of a compound SP3103 (Chinese name: 7-methoxy-4,5-dioxo-4,5-dihydro-1H-pyrrolo[2,3-f]quinone-9-carboxylic acid)

Dimethyl sulfate (1.26 g, 0.01 mole) was added into a reaction bottle first and heated to 80°C, and then a raw material SP3101 (3.1 g, 0.01 mole) was added in batches. After a complete reaction of the raw material monitored by thin layer chromatography (TLC), a certain amount of water was added into a reaction solution at 0°C, an ammonia water solution was slowly added, and a sodium bicarbonate solution was added dropwise to make the pH of the reaction solution reach about 4, and then, suction filtration was performed to obtain a product SP3103.

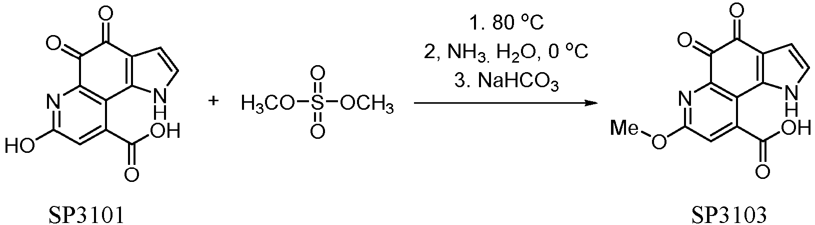

According to detection by mass spectrometry-electrospray in a positive ion mode, the sample has an m/z value of 273.1 (M+1)+, proving that the compound has a free molecular weight of 272. Nuclear magnetic resonance spectra are as shown in Table 3.

**Table 3 Attribution of hydrogen spectra and carbon spectra**

| Number | ¹H NMR | ¹³C NMR |
|---|---|---|
| 1 | 3.80, 3H, s | 54.1 |
| 2 | 5.0, 1H, s | / |
| 3 | 6.40 d, 1H, *j*=7.5 | 104.4 |
| 4 | 6.95 d, 1H, *j*=7.5 | 118.3 |
| 5 | 7.82, 1H, s | 112.9 |
| 6 | 11.0, 1H, s | / |
| 7 | / | 115.0 |
| 8 | / | 122.7 |
| 9 | / | 125.2 |
| 10 | / | 135.7 |
| 11 | / | 144.2 |
| 12 | / | 162.4 |
| 13 | / | 167.7 |
| 14 | / | 173.5 |
| 15 | / | 176.7 |

The sample was dissolved in DMSO-d6.

### Activity Test 1: Inflammatory pain model and study on analgesic effects of drugs

4 SD rats with a body weight of 200-250 g were used as a blank control (NC), and normal saline was injected into the soles of the left feet of the rats. Inflammatory pain models of 18 rats were constructed, that is to say, 100 µL of a complete Freund's adjuvant was injected into soles of the left feet of the rats, mechanical pain thresholds of the left feet were detected by an ALMEMO2490 data collector 24 hours later, 2 rats with a pain threshold of greater than 15 were abandoned, and the other 16 rats were randomly divided into 4 groups with 4 rats in each group and then administered with water as a positive control (PC), SP3101 (20 mg/kg), PQQ (20 mg/kg) and ibuprofen (25 mg/kg/day) by gavage, respectively.

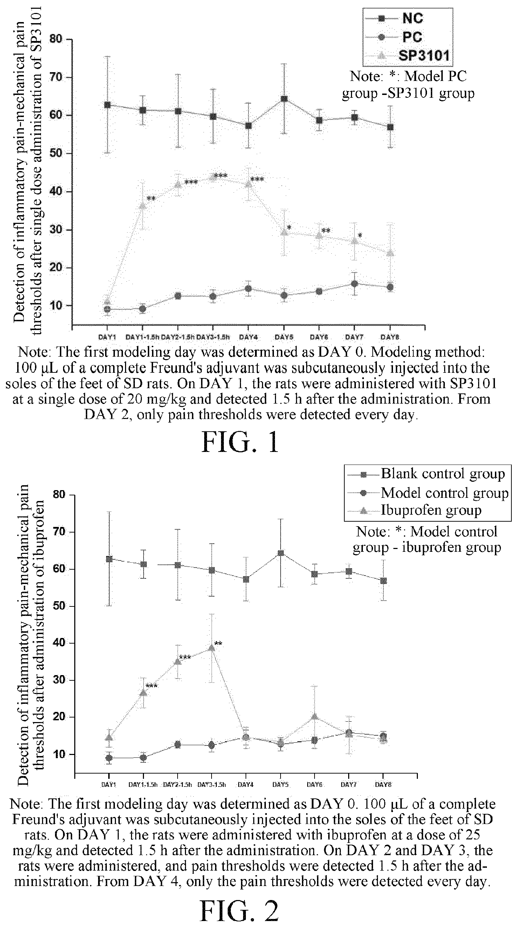

Results are as shown in FIG. 1 to FIG. 3.

FIG. 1 shows an analgesic effect of SP3101 on rats after single oral administration at a dose of 20 mg/kg. After single oral administration for 1.5 hours, an obvious analgesic effect is achieved, and the effect lasts for 72 hours (day 1-4). On day 5-7, the analgesic effect is reduced. However, compared with a model group (PC), obvious differences are still achieved (* indicates p<0.05, ** indicates p< 0.01, *** indicates p<0.001, and expressions are the same below).

FIG. 2 shows an analgesic effect of ibuprofen on rats after continuous oral administration at a dose of 25 mg/kg. The ibuprofen was administered once a day at a dose of 25 mg per kg of the body weight and continuously administered orally for 3 days, and the pain thresholds were detected 1.5 hours after each administration. Similar to SP3101, the ibuprofen has a rapid effect. The ibuprofen can achieve an obvious analgesic effect 1.5 hours after oral administration, can achieve an obvious analgesic effect after continuous administration on day 1/2/3, has no analgesic effect before administration (not shown with data), and has no analgesic effect on the fourth day without administration.

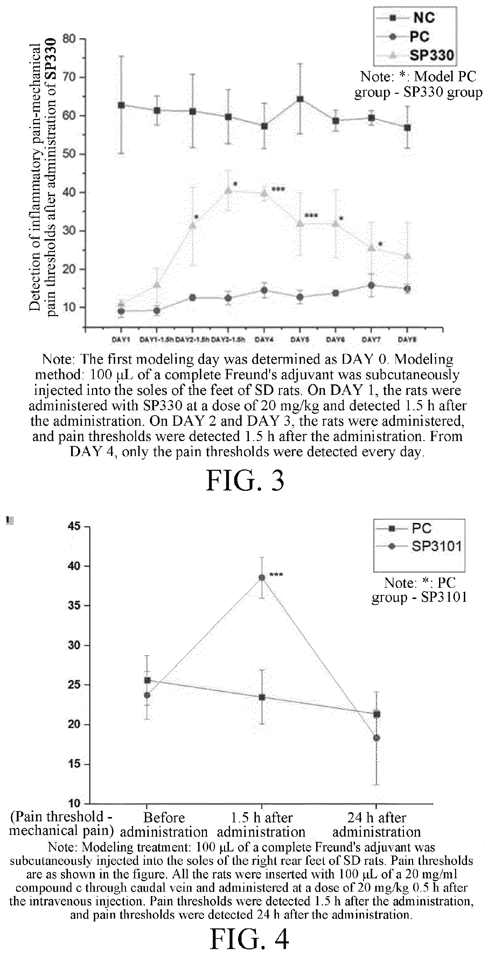

FIG. 3 shows an analgesic effect of SP330 (namely PQQ) on rats after continuous oral administration at a dose of 20 mg/kg/day for three days. No analgesic effect is achieved 1.5 hours after oral administration on the first day, and an obvious analgesic effect is achieved on day 2/3/4/5/6/7.

The results show that the compound SP3101 of the present invention has a rapid analgesic effect and a long-acting analgesic effect. In addition, the inventor has found that the compound SP3102 and the compound SP3103 also have similar effects.

### Activity Test 2: Study on an action mechanism of a long-acting analgesic effect

Similar to the modeling method in Activity Test 1, 100 µL of a complete Freund's adjuvant was injected into the soles of the left feet of SD rats with a body weight of 200-250 g, mechanical pain thresholds of the left feet were detected by an ALMEMO2490 data collector 24 hours later, and the rats with a pain threshold of greater than 15 were abandoned. On the second day of modeling, 100 µL of a compound c as an AMPK inhibitor (Dorsomorphin, CAS No.866405-64-3, CC, 0.4 mg/ml) was injected into the caudal vein of each rat, the rats were orally administered with SP3101 (20 mg/kg) half an hour later, and the pain thresholds were detected after 1.5 hours and 24 hours, respectively.

Results are as shown in FIG. 4. A short-acting (1.5 hours) analgesic effect of the SP3101 is not affected while AMPK is inhibited, but a long-acting (24 hours) analgesic effect of the SP3101 is eliminated.

### Activity Test 3: AMPK agonist

RAW264.7 cells were inoculated into two six-well plates and enabled to grow to a density of about 90%, and SP3101 and SP3102 of different concentrations were added, respectively. 24 hours later, the cells were lysed with a radio immunoprecipitation assay (RIPA) lysis buffer, and the expression of proteins was detected by protein hybridization.

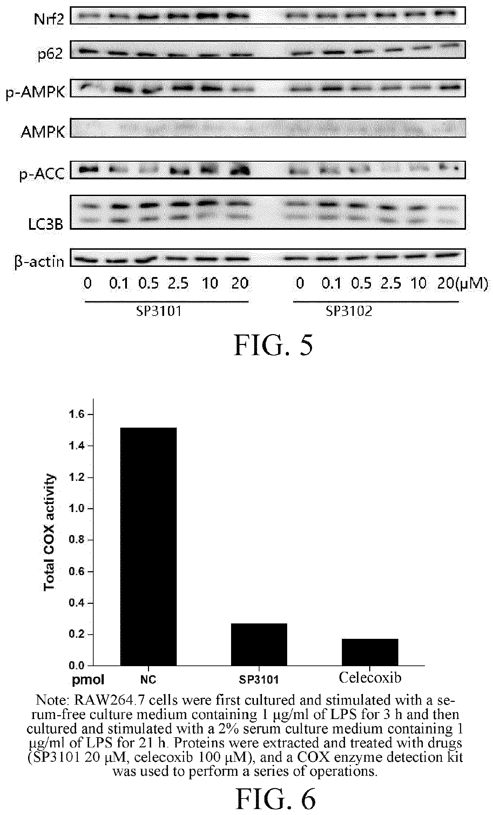

Phosphorylation of serine at the 127^{th} site of AMPK is used as an activation marker. Results are as shown in FIG. 5. The SP3101 and the SP3102 at a concentration of 0.1 µM can obviously activate the AMPK (the expression of p-AMPK is increased), while the expression of an antioxidant transcription factor (Nrf2) is increased in a dose-dependent manner, and the expression of an autophagy related protein (LC3B) is also increased. It can be seen that the compounds, represented by the SP3101 and the SP3102, of the present invention are used as an AMPK agonist.

### Activity Test 4: Inhibitor of Cox-2

### 1. Sample preparation

(1) RAW264.7 cells were resuscitated, enabled to grow to a density of about 100% and washed with phosphate buffered saline (PBS) for two times.
(2) The cells were cultured in a serum-free DMEM culture medium containing 1 µg/ml of lipopolysaccharide (LPS) for 3 hours and then cultured in a 2% serum DMEM culture medium containing 1 µg/ml of LPS for 21 hours.
(3) 1,110 µL of a cell lysis solution was prepared to blow at the cells, the cells were treated in an ice bath and shaken on a shaker for 15 minutes, a centrifuge was opened, and the temperature was lowered.
(4) Large dishes were scraped with the tip of a pipette, and treated solutions were sucked and added into two EP tubes, respectively. Then, the treated solutions were centrifuged at 12,000 r at 4°C for 15 minutes and stored on ice.

### 2. Detection of the activity of COX

The activity of an epoxidase (COX) was detected by an abcam kit (ab204699 cox activity assay kit, fluorometric) according to operations in an instruction. Finally, fluorescence detection was performed at an Ex/Em value of 535/587 nm.

Results are as shown in FIG. 6. The results show that SP3101 has a similar effect of inhibiting the activity of cox-2 as a positive control (celecoxib).

### Activity Test 5: Effect of SP3101 on inhibiting inflammation

As viral pneumonia of C57BL/B6 mice can be induced by nasal dropping of an MHV-A59 virus, the ability of SP3101 to inhibit virus-induced lung inflammation of mice is studied using such model.

An MHV-A59 virus strain was purchased from Suzhou Xishan Biotechnology Co., Ltd. at a PFU/ml of 1.4×10⁷.

6-week-old mice were divided into 3 groups with 8 mice in each group. After anesthesia, the mice in a blank control group were administered with normal saline through the noses, and viral pneumonia models of the other two groups were constructed as follows: 10 µL of an MHV was injected into the nose of each mouse at a PFU of 1.4×10⁵ with 5 µL into the left nostril and the right nostril, respectively, and about 3-6 hours later, the mice woke up. The mice in a model treatment group were administered with an SP3101 aqueous solution by gavage at a dose of 20 mg/kg body weight/day (mg/kg/day).

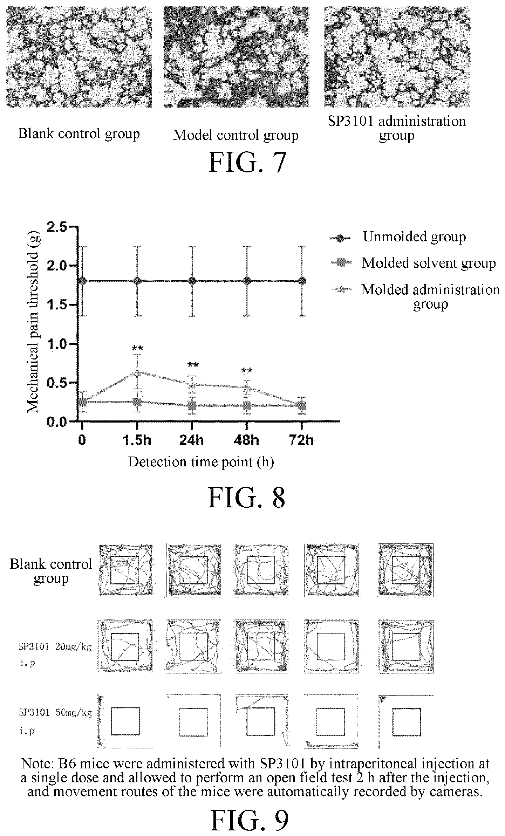

After continuous administration for 5 days, the mice were anesthetized and dissected 4 hours after the last administration, and the lungs were taken out, fixed with paraformaldehyde and stored for hematoxylin-eosin (HE) section staining. Pathological sections are as shown in FIG. 7. The SP3101 has an obvious effect of inhibiting virus-induced lung inflammation.

Activity Test 6: Neuropathic pain and study on an analgesic effect of a drug

A CCI model was used for realizing neuropathic pain. 6- to 8-week-old B6 mice were anesthetized, shaved from the roots of the left legs to parts about 1-2 mm higher than the horizontal lines of the tail root bones, and cut horizontally to obtain small wounds. Muscles were bluntly separated by hemostatic forceps, the sciatic nerves were found and picked out with elbow tweezers, and 4-0 chromium-containing catgut sutures soaked in sterile normal saline in advance were picked out with another elbow tweezer. Three catgut sutures were penetrated through the sciatic nerve of each mouse and ligatured in an upper part, a middle part and a lower part at a distance of 1 mm, respectively at an appropriate intensity enabling surrounding muscles to have small and transient twitches. Then, the sciatic nerves were placed under the muscles, and the wounds were sutured. The wounds were disinfected with an iodine tincture and observed.

On day 7 after surgery, mechanical pain thresholds of the mice were detected by using Von Frey fibrils. The mice were placed on a transparent sole test platform made from organic glass for adaptation for 20 minutes first, and then the forefeet (namely, right rear feet) on a modeling side of the mice were vertically stimulated with a series of Von Frey fibrils. The mice were stimulated at a lower intensity first and then stimulated at a higher intensity when positive responses were not induced by the lower intensity, where the positive responses were shown as rapid foot retractions. Then, the mice were stimulated again at an adjacent lower intensity, and the intensity interval of first positive responses and negative responses was recorded. Successful molding was indicated when a positive response value is less than 0.4 g.

The test mice were divided into three groups: an unmolded group, a molded solvent group and a molded administration group. 0.5% methylcellulose was used as a solvent, SP3101 was used as a drug and administered at a dose of 40 mg/kg according to the body weight each time, and the pain thresholds were detected 1.5, 24, 48 and 72 hours after administration. Results are as shown in FIG. 8. The SP3101 has an obvious analgesic effect 1.5, 24 and 48 hours after administration by gavage (**p<0.01).

Activity Test 7: Sedation test of SP3101

A total of 15 12-week-old male B6 mice were selected and allowed to adapt to the environment for three days. The mice were randomly divided into 3 groups including: (1) a blank control group (n=5), (2) a low-dose SP3101 group at a dose of 20 mg/kg (n=5) and (3) a high-dose SP3101 group at a dose of 50 mg/kg (n=5). The mice were administered by intraperitoneal injection at a single dose according to the groups, where the mice in the blank control group were administered with an equal volume of normal saline by intraperitoneal injection. 2 hours after the administration, the mice were traced by a video tracking and detection system in an open field test (OFT), followed by behavioral evaluation.

Results are as shown in FIG. 9. After the intraperitoneal injection, the SP3101 obviously reduces movement of the mice in an open field, thereby having an obvious sedative effect.

Activity Test 8: Test effects of SP3101 using an oxaliplatin chemotherapy pain model

A total of 30 10-week-old wild male C57LB6 mice were selected.

On DAY 0, the mice were allowed to adapt to the environment for 7 days first, basic mechanical pain thresholds were detected and were basically about 1-1.4 g, and a total of 30 mice were used in this test without abnormal mice.

On DAYS 1-5, the mice were randomly divided into 3 groups including a blank control group (n=10), a model control group (n=10) and an SP3101 group (40 mg/kg, n=10). The mice in the SP3101 group were administrated with an SP3101 aqueous solution by gavage, the mice in the model control group were administrated with double distilled water by gavage, and the mice in the model control group and the SP3101 group were administrated with oxaliplatin (3 mg/kg) by intraperitoneal injection 0.5 hour later. The same operations were continuously performed for 5 days. On DAY 5, the pain thresholds were detected by using Von Frey fibrils.

On DAYS 6-10, the mice were only administered with SP3101 and water by gavage.

On DAYS 11-15, the operations on DAYS 1-5 were repeated, and on DAY 11, the pain thresholds were detected by using Von Frey fibrils.

On DAYS 16-20, the mice were only observed, and on DAY 16 and DAY 20, the mechanical pain thresholds were detected.

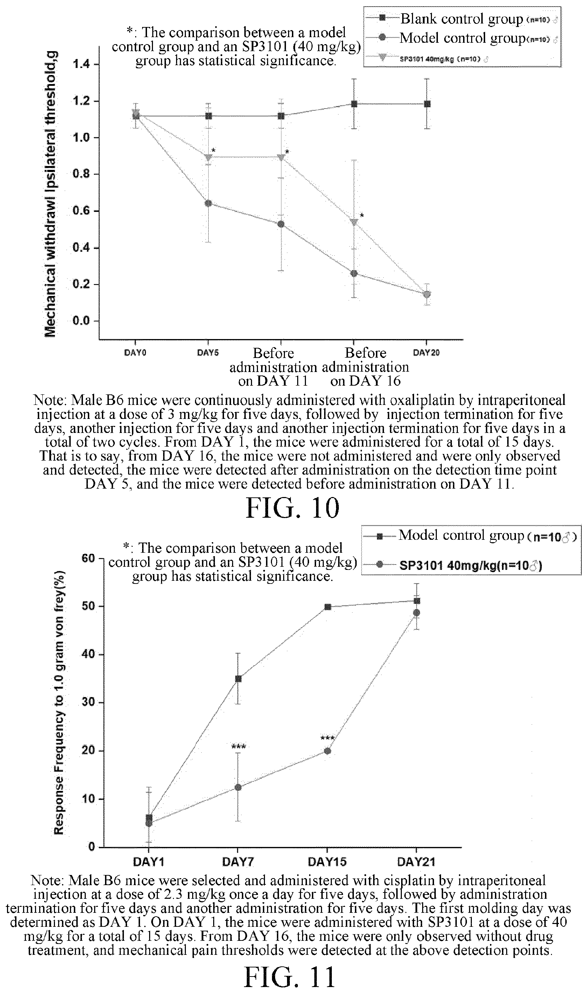

Results are as shown in FIG. 10. The SP3101 can obviously prevent oxaliplatin-induced hyperalgesia during administration and obviously improve the pain thresholds of the model mice.

Activity Test 9: Effects of SP3101 using a cisplatin chemotherapy pain model

20 12-week-old male B6 mice were selected and allowed to adapt to the environment for three days.

On DAY 1, basic mechanical pain thresholds were detected by using Von Frey fibrils, the mice with abnormal pain thresholds were abandoned (no abnormal mice were found in this batch), and the pain thresholds were basically about 1.4-2.0 g. According to the basic pain thresholds, a pain threshold of 0.4 g was used in this test, and a pain threshold of 1.0 g was used in subsequent detection. Behaviors such as foot lifting and foot licking were identified as positive responses. According to statistics of the proportion of positive responses of the mice using the Von Frey fibrils at the two corresponding thresholds, whether SP3101 had a preventive effect on cisplatin-induced hyperalgesia after administration by gavage was determined.

On DAYS 1-5, after the basic mechanical pain thresholds were detected, the mice were randomly divided into the following groups: (1) a model control group (n=10) and (2) an SP3101 group (n=10) administered by gavage at a dose of 40 mg/kg/day, where the mice in the model control group were administered with double distilled water by gavage, and the mice in the two groups were administered with cisplatin prepared with normal saline (2.3 mg/kg/day) by intraperitoneal injection 0.5 hour later.

On DAYS 6-10, the mice were only administered with SP3101 and water by gavage, and on DAY 7, the mechanical pain thresholds were detected.

On DAYS 11-15, the operations on DAYS 1-5 were repeated, and on DAY 15, the mechanical pain thresholds were detected.

On DAYS 16-21, the mice were only observed, and on DAY 21, the mechanical pain thresholds were detected.

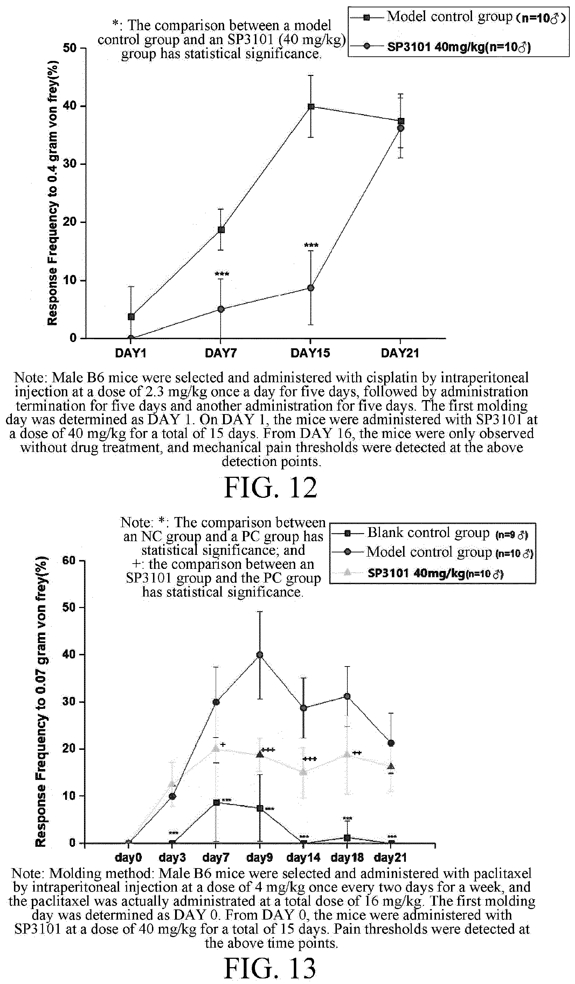

Results are as shown in FIG. 11 and FIG. 12. The SP3101 has an obvious effect of alleviating the cisplatin-induced hyperalgesia.

Activity Test 10: Data of SP3101 using a paclitaxel chemotherapy pain model

A total of 30 8- to 10-week-old male B6 mice were selected and allowed to adapt to the environment for three days.

On DAY 0, mechanical pain thresholds were detected by using Von Frey fibrils, the mice with abnormal pain thresholds were abandoned, and the basic pain thresholds were basically about 0.6-1.0 g. According to the basic pain thresholds, a pain threshold of 0.07 g was used in this test, and a pain threshold of 0.4 g was used in subsequent detection. Behaviors such as foot lifting and foot licking were identified as positive responses. According to statistics of the proportion of positive responses of the mice using the Von Frey fibrils at the two corresponding thresholds, whether SP3101 had a preventive effect on paclitaxel-induced hyperalgesia after administration by gavage was determined. The mice were randomly divided into the following groups: (1) a model control group (PC, n=10), (2) an SP3101 group administered at a dose of 40 mg/kg (n=10) by gavage and (3) a blank control group (NC). The mice in the model control group were administrated with double distilled water by gavage, the mice in the model control group and the SP3101 group were administrated with paclitaxel (4 mg/kg) by intraperitoneal injection 0.5 hour later, and the mice in the blank control group were administrated with normal saline by intraperitoneal injection. According to a paclitaxel chemotherapy pain modeling method, the mice were administrated with paclitaxel (4 mg/kg) by intraperitoneal injection every other day for 4 times in 7 days, and the paclitaxel was actually administrated at a total dose of 16 mg/kg. The first modeling day was determined as DAY 0. From DAY 0, the mice were administrated with SP3101 by gavage once a day for a total of 15 days. After DAY 14, the mice were only observed. On DAY 0, 3, 7, 9, 14, 18 and 21, the mechanical pain thresholds were detected.

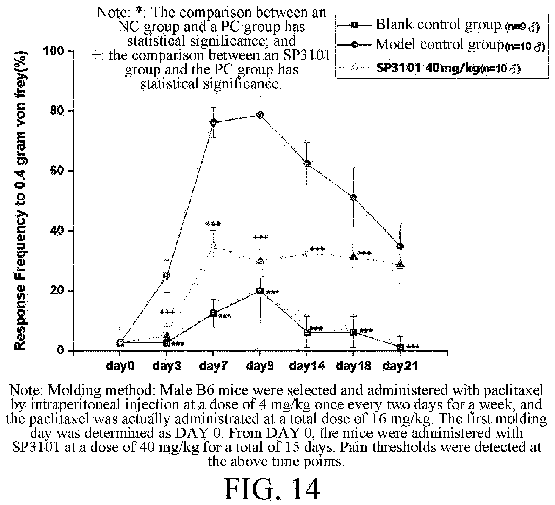

Results are as shown in FIG. 13 and FIG. 14. Through comparison of the SP3101 group and the model control group, the proportions of pain threshold responses in the two groups were both obviously decreased, indicating that the SP3101 has an obvious effect of alleviating the paclitaxel-induced hyperalgesia.

## Claims

1. A compound of a formula (I) or a pharmaceutically acceptable salt, ester, stereoisomer or solvent compound thereof, 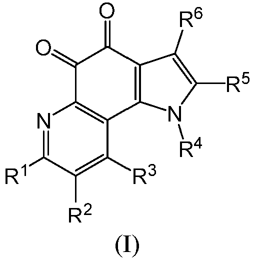 wherein
R¹ is selected from -OH, -OR, -OC(O)R, -NH₂, -NHR, -SH or -SR;
R² is selected from H;
R³ is selected from H or -COOH;
R⁴ is selected from H;
R⁵ is selected from H or -COOH;
R⁶ is selected from H;
R is C₁-C₆ alkyl; and preferably, the R is C₁-C₃ alkyl.

2. A compound according to claim 1 of a formula (II) or a pharmaceutically acceptable salt, ester, stereoisomer or solvent compound thereof, 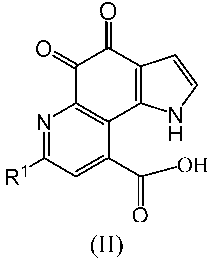 wherein R¹ is selected from -OH, -OR, -OC(O)R, -NH₂, -NHR, -SH or -SR; R is C₁-C₆ alkyl; and preferably, the R is C₁-C₃ alkyl.

3. Compounds or pharmaceutically acceptable salts, esters, stereoisomers or solvent compounds thereof shown as follows:

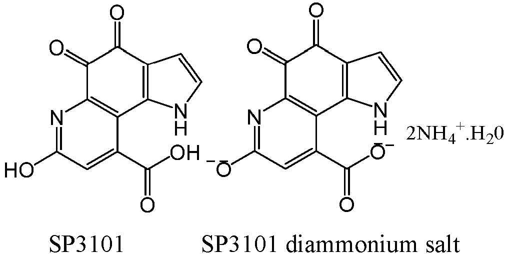

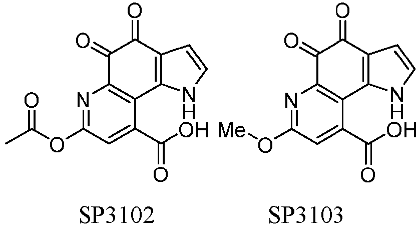

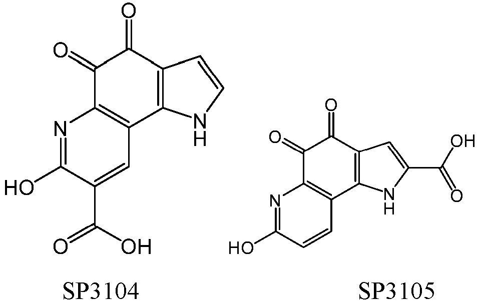

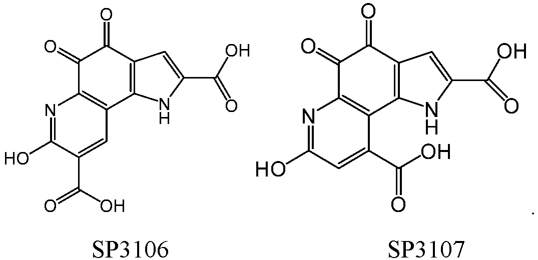

4. A method for preparing the compound of a formula (II):

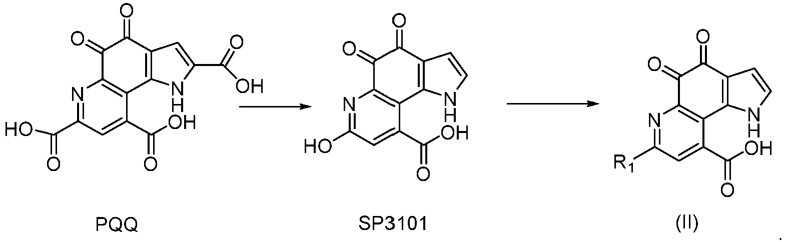

5. A pharmaceutical composition, comprising the compounds of any of claims 1-3 or pharmaceutically acceptable salts, esters, stereoisomers or solvent compounds thereof, and a pharmaceutically acceptable carrier.

6. The compound or a pharmaceutically acceptable salt, ester, stereoisomer or solvent compound thereof according to any one of claims 1 to 3 for use as an AMPK agonist.

7. The compound or a pharmaceutically acceptable salt, ester, stereoisomer or solvent compound thereof according to any one of claims 1 to 3 for use as an inhibitor of a Cox-2 enzyme.

8. The compound or a pharmaceutically acceptable salt, ester, stereoisomer or solvent compound thereof according to any one of claims 1 to 3 for us as analgesic drugs.

9. The compound or a pharmaceutically acceptable salt, ester, stereoisomer or solvent compound thereof according to any one of claims 1 to 3 for use in prevention and treatment of inflammatory diseases.

10. The compound or a pharmaceutically acceptable salt, ester, stereoisomer or solvent compound thereof according to any one of claims 1 to 3 for use as anti-tumor drugs; or for use in the chemotherapeutic treatment, for use as chemical substances or drugs in prevention and treatment of tumors caused by cytotoxins, in prevention and treatment of various liver injuries and hepatic insufficiency caused by chronic hepatitis, and in prevention and treatment of myocardial infarction injuries and viral myocarditis; or for use in prevention and treatment of neurodegeneration, change diseases, epilepsy and migraine.

11. The compound or a pharmaceutically acceptable salt, ester, stereoisomer or solvent compound thereof according to any one of claims 1 to 3 for use in prevention and treatment of peripheral neuropathy caused by chemotherapy.

12. The compound or a pharmaceutically acceptable salt, ester, stereoisomer or solvent compound thereof according to any one of claims 1 to 3 for use in prevention and treatment of depression, and sedative or hypnotic drugs.

## Patentansprüche

1. Eine Verbindung mit der Formel (I) oder ein pharmazeutisch verträgliches Salz, ein Ester, ein Stereoisomer oder eine Lösungsmittelverbindung davon, 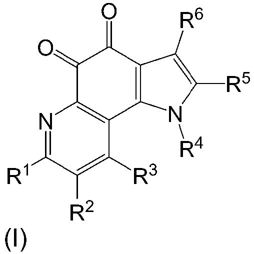 wobei
R¹ aus -OH, -OR, -OC(O)R, -NH₂, -NHR, -SH oder -SR ausgewählt ist;
R² aus H ausgewählt ist;
R³ aus H oder -COOH ausgewählt ist;
R⁴ aus H ausgewählt ist;
R⁵ aus H oder -COOH ausgewählt ist;
R⁶ aus H ausgewählt ist;
R C₁-C₆-Alkyl ist; und vorzugsweise R C₁-C₃-Alkyl ist.

2. Verbindung gemäß Anspruch 1 mit der Formel (II) oder ein pharmazeutisch verträgliches Salz, ein Ester, ein Stereoisomer oder eine Lösungsmittelverbindung davon, 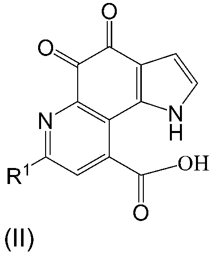 wobei R¹ aus -OH, -OR, -OC(O)R, -NH₂, -NHR, -SH oder -SR ausgewählt ist; R C₁-C₆-Alkyl ist; und vorzugsweise R C₁-C₃-Alkyl ist.

3. Verbindungen oder pharmazeutisch verträgliche Salze, Ester, Stereoisomere oder Lösungsmittelverbindungen davon, die wie folgt dargestellt sind:

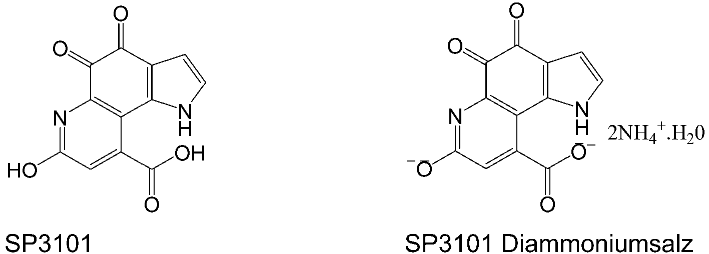

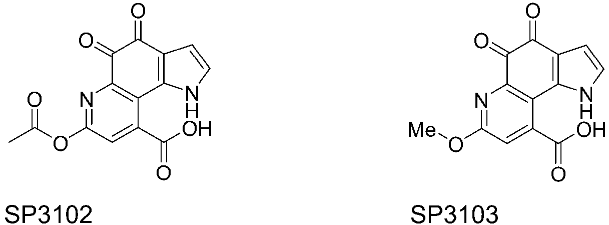

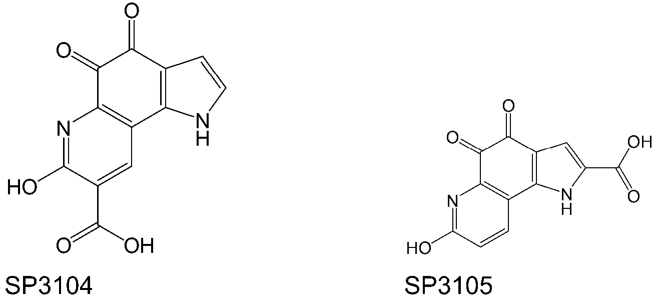

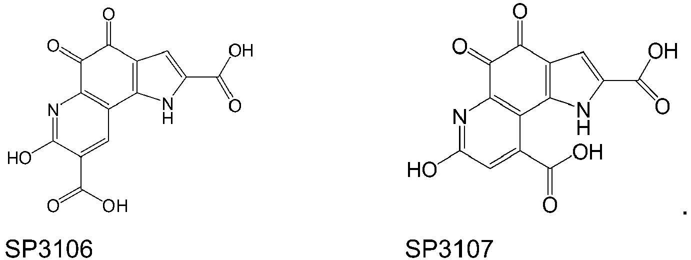

4. Verfahren zur Herstellung der Verbindung mit der Formel (II):

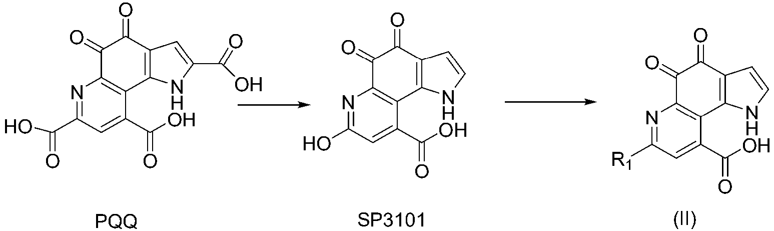

5. Pharmazeutische Zusammensetzung, umfassend die Verbindungen gemäß einem der Ansprüche 1 bis 3 oder pharmazeutisch verträgliche Salze, Ester, Stereoisomere oder Lösungsmittelverbindungen davon sowie einen pharmazeutisch verträglichen Träger.

6. Verbindung oder ein pharmazeutisch verträgliches Salz, ein Ester, ein Stereoisomer oder eine Lösungsmittelverbindung davon gemäß einem der Ansprüche 1 bis 3 zur Verwendung als AMPK-Agonist.

7. Verbindung oder ein pharmazeutisch verträgliches Salz, ein Ester, ein Stereoisomer oder eine Lösungsmittelverbindung davon gemäß einem der Ansprüche 1 bis 3 zur Verwendung als Inhibitor eines Cox-2-Enzyms.

8. Verbindung oder ein pharmazeutisch verträgliches Salz, ein Ester, ein Stereoisomer oder eine Lösungsmittelverbindung davon gemäß einem der Ansprüche 1 bis 3 zur Verwendung als Analgetikum.

9. Verbindung oder ein pharmazeutisch verträgliches Salz, ein Ester, ein Stereoisomer oder eine Lösungsmittelverbindung davon gemäß einem der Ansprüche 1 bis 3 zur Verwendung bei der Vorbeugung und Behandlung von Entzündungskrankheiten.

10. Verbindung oder ein pharmazeutisch verträgliches Salz, ein Ester, ein Stereoisomer oder eine Lösungsmittelverbindung davon gemäß einem der Ansprüche 1 bis 3 zur Verwendung als Antitumormittel; oder zur Verwendung bei der chemotherapeutischen Behandlung, zur Verwendung als chemische Substanzen oder Arzneimittel bei der Vorbeugung und Behandlung von durch Zytotoxinen verursachten Tumoren, zur Vorbeugung und Behandlung verschiedener Leberschäden und Leberinsuffizienz, die durch chronische Hepatitis verursacht werden, sowie zur Vorbeugung und Behandlung von Myokardinfarkt-Schäden und viraler Myokarditis; oder zur Verwendung bei der Vorbeugung und Behandlung von Neurodegeneration, Neuropathien, Epilepsie und Migräne.

11. Verbindung oder ein pharmazeutisch verträgliches Salz, ein Ester, ein Stereoisomer oder eine Lösungsmittelverbindung davon gemäß einem der Ansprüche 1 bis 3 zur Verwendung bei der Vorbeugung und Behandlung von durch Chemotherapie verursachter peripherer Neuropathie.

12. Verbindung oder ein pharmazeutisch verträgliches Salz, ein Ester, ein Stereoisomer oder eine Lösungsmittelverbindung davon gemäß einem der Ansprüche 1 bis 3 zur Verwendung bei der Vorbeugung und Behandlung von Depressionen sowie als Beruhigungs- oder Schlafmittel.

## Revendications

1. Composé de formule (I) ou sel, ester, stéréoisomère ou composé solvant pharmaceutiquement acceptable de celui-ci, 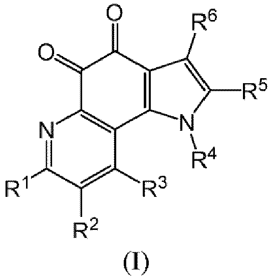 dans laquelle
R¹ est choisi parmi -OH, -OR, -OC(O)R, -NH₂, -NHR, -SH ou -SR ;
R² est choisi parmi H ;
R³ est choisi parmi H ou -COOH ;
R⁴ est choisi parmi H ;
R⁵ est choisi parmi H ou -COOH ;
R⁶ est choisi parmi H ;
R est un alkyle en C₁-C₆ ; et de préférence, R est un alkyle en C₁-C₃.

2. Composé selon la revendication 1 de formule (II) ou sel, ester, stéréoisomère ou composé solvant pharmaceutiquement acceptable de celui-ci, 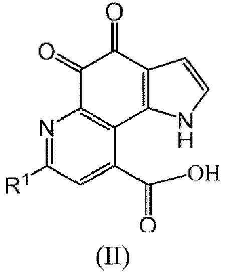 dans laquelle R¹ est choisi parmi -OH, -OR, -OC(O)R, -NH₂, -NHR, -SH ou -SR ; R est un alkyle en C₁-C₆ ; et de préférence, R est un alkyle en C₁-C₃.

3. Composés ou sels, esters, stéréoisomères ou composés solvants pharmaceutiquement acceptables de ceux-ci représentés comme suit :

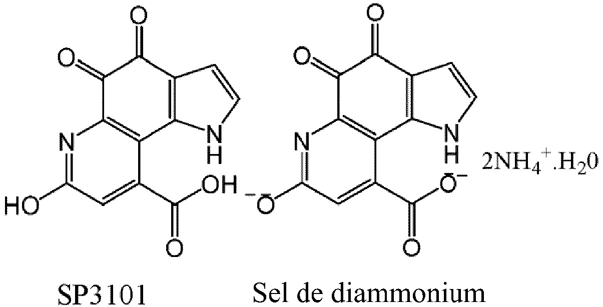

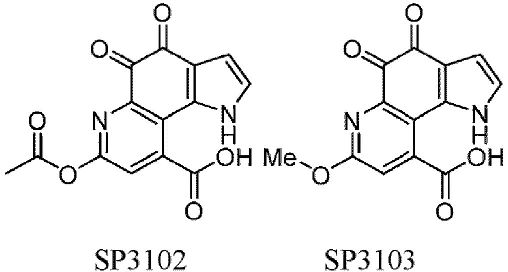

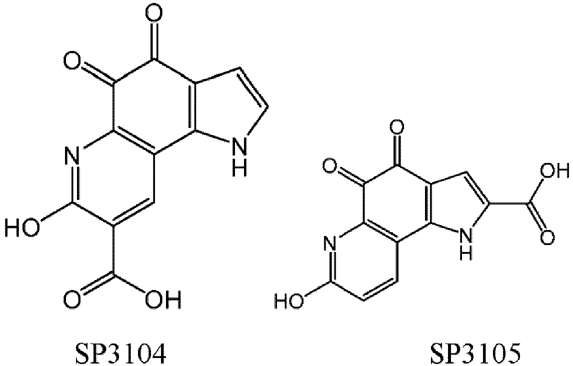

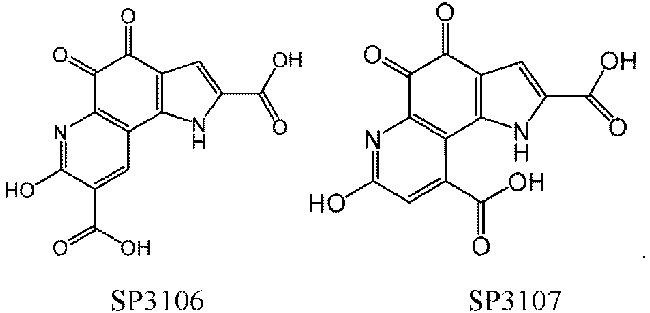

4. Procédé de préparation du composé de formule (II) :

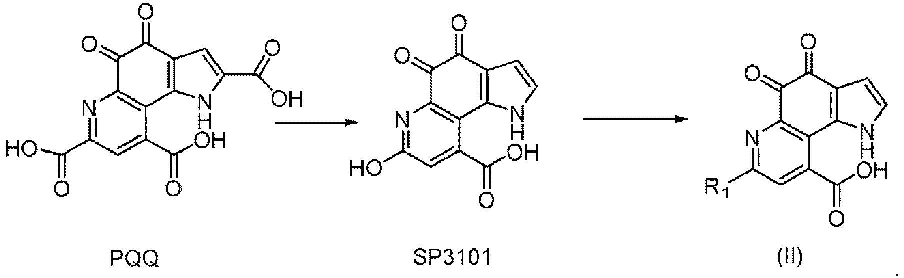

5. Composition pharmaceutique, comprenant les composés selon l'une quelconque des revendications 1 à 3 ou des sels, esters, stéréoisomères ou composés solvants pharmaceutiquement acceptables de ceux-ci, et un support pharmaceutiquement acceptable.

6. Composé ou sel, ester, stéréoisomère ou composé solvant pharmaceutiquement acceptable de celui-ci selon l'une quelconque des revendications 1 à 3 pour une utilisation en tant qu'agoniste de l'AMPK.

7. Composé ou sel, ester, stéréoisomère ou composé solvant pharmaceutiquement acceptable de celui-ci selon l'une quelconque des revendications 1 à 3 pour une utilisation en tant qu'inhibiteur d'une enzyme Cox-2.

8. Composé ou sel, ester, stéréoisomère ou composé solvant pharmaceutiquement acceptable de celui-ci selon l'une quelconque des revendications 1 à 3 pour une utilisation en tant que médicaments analgésiques.

9. Composé ou sel, ester, stéréoisomère ou composé solvant pharmaceutiquement acceptable de celui-ci selon l'une quelconque des revendications 1 à 3 pour une utilisation dans la prévention et le traitement de maladies inflammatoires.

10. Composé ou sel, ester, stéréoisomère ou composé solvant pharmaceutiquement acceptable de celui-ci selon l'une quelconque des revendications 1 à 3 pour une utilisation en tant que médicaments antitumoraux ; ou pour une utilisation dans le traitement chimiothérapeutique, pour une utilisation en tant que substances chimiques ou médicaments dans la prévention et le traitement de tumeurs provoquées par des cytotoxines, dans la prévention et le traitement de diverses lésions hépatiques et de l'insuffisance hépatique du fait d'une hépatite chronique, et dans la prévention et le traitement de lésions dues à l'infarctus du myocarde et de la myocardite virale ; ou pour une utilisation dans la prévention et le traitement de la neurodégénérescence, de maladies de changement, de l'épilepsie et de la migraine.

11. Composé ou sel, ester, stéréoisomère ou composé solvant pharmaceutiquement acceptable de celui-ci selon l'une quelconque des revendications 1 à 3 pour une utilisation dans la prévention et le traitement de la neuropathie périphérique provoquée par une chimiothérapie.

12. Composé ou sel, ester, stéréoisomère ou composé solvant pharmaceutiquement acceptable de celui-ci selon l'une quelconque des revendications 1 à 3 pour une utilisation dans la prévention et le traitement de la dépression, et médicaments sédatifs ou hypnotiques.
